# EUROPEAN PATENT APPLICATION

(11) **EP 4 843 531 A1**
(43) Date of publication of application: **25.11.2026**
(21) Application number: 25742121.4
(22) Date of filing: 08.01.2025
(51) Int. Cl.: A61C 7/00, A61C 7/08, A61C 9/00, G06T 7/00, G16H 30/00, A61C 19/04, G06Q 50/04, G06F 17/00, B33Y 10/00, B33Y 50/00

(54) **METHOD AND DEVICE FOR MANUFACTURING TRANSPARENT ORTHODONTIC DEVICE, AND RECORDING MEDIUM**

(30) Priority: 16.01.2024 KR 20240006564
(71) Applicant: Osstemimplant Co., Ltd., Gangseo-gu Seoul 07789 (KR)
(72) Inventor: KIM, Sang Chul, Seoul 03388 (KR); LEE, Kyung Woo, Bucheon-si, Gyeonggi-do 14663 (KR); SEO, Jin Woo, Goyang-si, Gyeonggi-do 10310 (KR); CHOI, Kyoo Ok, Seoul 07789 (KR)
(74) Representative: Angerhausen, Christoph
(86) International application number: PCT/KR2025/000418
(87) International publication number: WO 2025/155025

(57) **Abstract**

Disclosed are a method, a device for performing the method, and a recording medium, the method including: obtaining, by a processor, specification information on a plurality of standard pre-aligners generated based on statistical information; obtaining, by a receiver, oral information of a user; determining, by the processor, one of the plurality of standard pre-aligners based on the oral information and the specification information; obtaining, by the processor, a tooth model corresponding to the oral information; and obtaining, by the processor, a clear aligner corresponding to the tooth model by deforming the determined standard pre-aligner to correspond to the tooth model.

## Description

### TECHNICAL FIELD

The present disclosure relates to a technology for acquiring oral information of a user and obtaining a clear aligner for a tooth model corresponding to the oral information of the user.

### BACKGROUND ART

A clear aligner, which is also referred to as an aligner or by the trade name Invisalign, is formed in the shape of a mouthpiece made of a thin and transparent plastic material. The clear aligner can be worn on teeth to correct the teeth without using wires and brackets and provides a favorable aesthetic appearance, and thus has recently been widely used.

In this regard, orthodontic treatment using a clear aligner employs the property that teeth move when an external force is applied thereto, and is performed in a manner of gradually moving the teeth to desired positions. Orthodontic treatment may be classified into labial orthodontic treatment, lingual orthodontic treatment, clear aligner treatment, and the like. In the case of clear aligner treatment, tooth correction is enabled by inducing gradual movement of teeth using transparent plastic without attaching metal structures, such as brackets, to the oral cavity, and thus clear aligner treatment has recently been widely used in orthodontic treatment.

However, conventional methods of manufacturing a clear aligner currently use a clear aligner sheet. When a clear aligner is manufactured using a clear aligner sheet, a tooth model is printed using a 3D printer according to a treatment stage based on oral scanner data. Thereafter, the clear aligner is manufactured using the sheet and the tooth model in thermoforming equipment. Accordingly, the clear aligner is manufactured through a step of polishing a trim line for removing a remaining portion of the sheet left after the clear aligner is manufactured.

Such a manufacturing method has limitations in that, when the clear aligner is manufactured from the sheet, a remaining portion of the sheet must be cut off and discarded, and a long period of time is required to manufacture the clear aligner. In addition, when a three-dimensional clear aligner is manufactured from a two-dimensional sheet, the sheet is stretched, thereby causing a thickness variation. As a result, a force applied by the clear aligner may vary for each tooth, each tooth surface, and each patient.

Accordingly, there is a need for a technology relating to a method of manufacturing a clear aligner that prevents a change in thickness while manufacturing the clear aligner having a three-dimensional shape.

### DETAILED DESCRIPTION OF THE INVENTION

### TECHNICAL PROBLEM

An embodiment of the present disclosure is intended to solve the above-described problems of the related art, and to provide a method, device, and recording medium capable of reducing material consumption caused by manufacturing and shortening a working time in the process of manufacturing a clear aligner.

The technical problem to be solved is not limited to the above-described technical problem, and various other technical problems may be further included within a scope apparent to those skilled in the art.

### TECHNICAL SOLUTION

A method of manufacturing a clear aligner according to a first aspect of the present disclosure may include: obtaining, by a processor, specification information on a plurality of standard pre-aligners generated based on statistical information; obtaining, by a receiver, oral information of a user; determining, by the processor, one of the plurality of standard pre-aligners based on the oral information and the specification information; obtaining, by the processor, a tooth model corresponding to the oral information; and obtaining, by the processor, a clear aligner corresponding to the tooth model by deforming the determined standard pre-aligner to correspond to the tooth model.

In addition, obtaining the clear aligner may include: performing heat treatment and vacuum treatment in a state in which the determined standard pre-aligner and the tooth model are coupled to each other; and separating the clear aligner from the tooth model after performing the heat treatment and the vacuum treatment.

In addition, the specification information may include information on an arch length indicating an anteroposterior length of a dental arch, an arch width indicating a left-right width of the dental arch, and a tooth width indicating a width of a tooth.

In addition, the tooth width may include an anterior tooth width and a posterior tooth width.

In addition, determining one of the plurality of standard pre-aligners based on the oral information and the specification information may include: obtaining user arch length information, user arch width information, and user tooth width information for the user based on the oral information; and determining one of the plurality of standard pre-aligners based on the user arch length information, the user arch width information, and the user tooth width information.

In addition, determining one of the plurality of standard pre-aligners based on the user arch length information, the user arch width information, and the user tooth width information may include: determining, by the processor, a user arch type as one of a U shape, an O shape, and a V shape; and determining one of the plurality of standard pre-aligners based on the determined user arch type.

In addition, the method may further include generating a 3D image based on the statistical information and printing the plurality of standard pre-aligners through a 3D printer, and shapes of the plurality of standard pre-aligners may be formed based on 3D shapes of a plurality of standard tooth models determined based on the statistical information.

In addition, an internal space of the standard pre-aligner may be configured to define a clearance that is maintained even after the standard tooth model is coupled to the standard pre-aligner.

In addition, a shape of any one standard pre-aligner among the plurality of standard pre-aligners may be a shape corresponding to one of the plurality of standard tooth models.

A device for manufacturing a clear aligner according to a second aspect of the present disclosure may include: a receiver configured to obtain oral information of a user; and a processor configured to obtain specification information on a plurality of standard pre-aligners generated based on statistical information, determine one of the plurality of standard pre-aligners based on the oral information and the specification information, obtain a tooth model corresponding to the oral information, and obtain a clear aligner corresponding to the tooth model by deforming the determined standard pre-aligner to correspond to the tooth model.

In addition, the processor may control a vacuum heat treatment unit to perform heat treatment and vacuum treatment in a state in which the determined standard pre-aligner and the tooth model are coupled to each other, and may separate the clear aligner from the tooth model after performing the heat treatment and the vacuum treatment.

In addition, the specification information may include information on an arch length indicating an anteroposterior length of a dental arch, an arch width indicating a left-right width of the dental arch, and a tooth width indicating a width of a tooth, and the tooth width may include an anterior tooth width and a posterior tooth width.

In addition, the processor may obtain user arch length information, user arch width information, and user tooth width information for the user based on the oral information, and may determine one of the plurality of standard pre-aligners based on the user arch length information, the user arch width information, and the user tooth width information.

In addition, the processor may determine a user arch type as one of a U shape, an O shape, and a V shape, and may determine one of the plurality of standard pre-aligners based on the determined user arch type.

A third aspect of the present disclosure may provide a computer-readable recording medium having recorded thereon a program for executing the method according to the first aspect on a computer. Alternatively, a fourth aspect of the present disclosure may provide a computer program stored in a recording medium to implement the method according to the first aspect.

### ADVANTAGEOUS EFFECTS OF THE INVENTION

According to an embodiment, since a clear aligner is obtained by providing a customized ready-made three-dimensional shape for manufacturing the clear aligner without using a sheet, material consumption can be reduced in that there is no need to discard a remaining portion of a material after manufacturing.

In addition, since the clear aligner having a three-dimensional form is obtained from the three-dimensional shape by providing the customized ready-made three-dimensional shape for manufacturing the clear aligner, it is advantageous in that a change in thickness does not occur during the manufacturing process, and the manufacturing period can also be shortened.

The effects of the present disclosure are not limited to the above-described effects, and should be understood as including all effects that can be inferred from the detailed description of the present disclosure or the configurations of the disclosure described in the claims.

### BRIEF DESCRIPTION OF THE DRAWINGS

FIG. 1 is a block diagram illustrating an example of a configuration of a device according to an embodiment.
FIG. 2 is a flowchart illustrating respective steps in which a device obtains a clear aligner according to an embodiment.
FIG. 3 is a diagram for explaining an example in which a device obtains an arch length and an arch width for a plurality of standard pre-aligners according to an embodiment.
FIG. 4 is a diagram for explaining an example in which a device obtains tooth widths for a plurality of standard pre-aligners according to an embodiment.
FIG. 5 is a diagram for explaining an example in which a device determines one of a plurality of standard pre-aligners based on a user arch type according to an embodiment.
FIG. 6 is a diagram for explaining an example in which a clear aligner is obtained from a three-dimensional shape according to an embodiment.

### MODE FOR CARRYING OUT THE INVENTION

Terms used in the embodiments have been selected as general terms that are currently widely used as much as possible in consideration of functions, but may vary depending on an intention of a person skilled in the art, precedents, emergence of new technologies, and the like. In addition, in certain cases, terms may be arbitrarily selected by the applicant, and in such cases, meanings thereof will be described in detail in a corresponding portion of the description of the invention. Therefore, the terms used in the present disclosure should be defined based not merely on names of the terms, but on meanings of the terms and the overall content of the present disclosure.

Throughout the specification, when a certain portion is described as "including" a certain component, this means that the certain portion may further include another component, rather than excluding another component, unless otherwise stated. In addition, terms such as "... unit" described in the specification refer to units that process at least one function or operation, and may be implemented as hardware or software, or as a combination of hardware and software.

Hereinafter, embodiments of the present disclosure will be described in detail with reference to the accompanying drawings so that those skilled in the art to which the present disclosure pertains can easily carry out the present disclosure. However, the present disclosure may be implemented in various different forms and is not limited to the embodiments described herein.

Throughout the specification, the term "region" may be interpreted as a concept including both two dimensions and three dimensions.

Hereinafter, a plurality of embodiments will be described in detail with reference to the drawings.

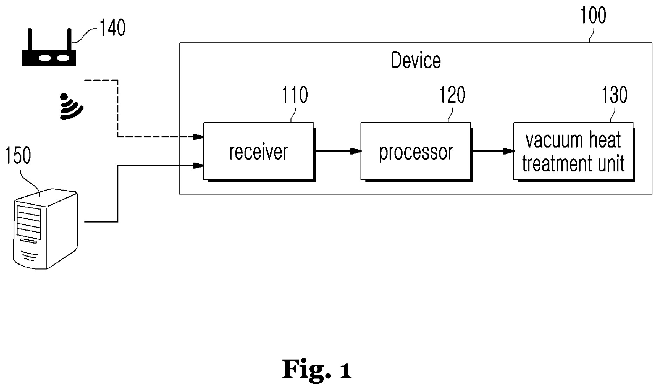

FIG. 1 is a block diagram illustrating an example of a configuration of a device 100 according to an embodiment.

Referring to FIG. 1, the device 100 may include a receiver 110, a processor 120, and a vacuum heat treatment unit 130.

It will be understood by those skilled in the art will understand that other general-purpose components may be further included in the device 100 in addition to the components illustrated in FIG. 1. For example, the device 100 may further include a memory (not shown). Alternatively, according to another embodiment, it will be understood by those skilled in the art will understand that some of the components illustrated in FIG. 1 may be omitted.

The receiver 110 according to an embodiment may obtain oral information of a user. For example, the receiver 110 may receive oral information or a 3D image of an oral cavity from an external device. However, the present embodiment is not limited thereto, and the device 100 may obtain oral information by obtaining a plurality of images corresponding to the oral cavity of the user.

The oral information may be generated based on various data obtained with respect to the oral cavity. For example, the oral information may be obtained based on scan data and/or CT data of the oral cavity. The CT data and the scan data may be received through a scanner or an external device. Referring to FIG. 1, a wireless terminal 140 or a wired terminal 150 may be an example of the external device.

The processor 120 according to an embodiment may obtain specification information on a plurality of standard pre-aligners generated based on statistical information. In addition, the processor 120 may determine one of the plurality of standard pre-aligners based on the oral information and the specification information. In addition, the processor 120 may obtain a tooth model corresponding to the oral information. In addition, the processor 120 may obtain a clear aligner corresponding to the tooth model by deforming the determined standard pre-aligner to correspond to the tooth model.

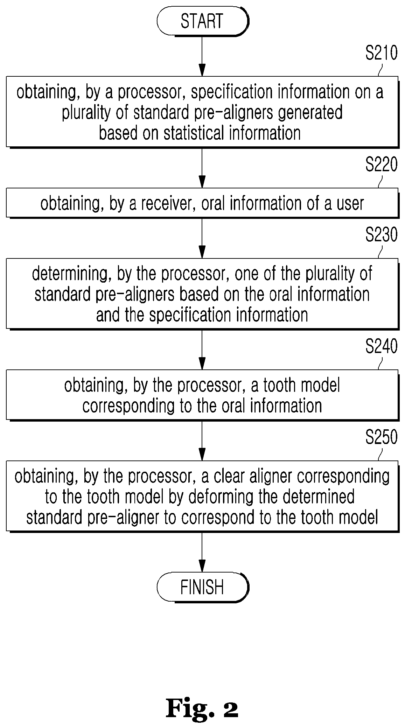

The embodiments described above with reference to FIG. 1 will be described in more detail with reference to FIGS. 2 to 6.

FIG. 2 is a flowchart illustrating respective steps in which the device 100 obtains a clear aligner according to an embodiment.

In operation S210, the device 100 according to an embodiment obtains specification information on a plurality of standard pre-aligners generated based on statistical information.

In an embodiment, the plurality of standard pre-aligners may be aligners generated based on statistical information on various tooth models obtained from an external device or an external server, and may include a plurality of preset aligners. That is, the plurality of standard pre-aligners may correspond to preformed transparent appliances. The device 100 may obtain, as the plurality of standard pre-aligners, a plurality of preformed transparent appliances that can be predicted to have standard shapes based on the statistical information.

The device 100 may obtain specification information on each of the plurality of standard pre-aligners. In an embodiment, the specification information may include information on an arch length indicating an anteroposterior length of a dental arch, an arch width indicating a left-right width of the dental arch, and a tooth width indicating a width of a tooth. Accordingly, the device 100 may obtain an arch length, an arch width, and a tooth width for each of the plurality of standard pre-aligners indicating the plurality of preset aligners.

In operation S220, the device 100 according to an embodiment obtains oral information of a user.

In an embodiment, the oral information may refer to information on a tooth shape, a dentition, and the like of a user who intends to wear a clear aligner. For example, the device 100 may obtain, as the oral information, 3D data generated using scan data or CT data for a plurality of teeth of the user. In addition, the device 100 may generate the oral information by obtaining scan data or CT data for the plurality of teeth, or may obtain the oral information from an external device.

In operation S230, the device 100 according to an embodiment determines one of the plurality of standard pre-aligners based on the oral information and the specification information.

In an embodiment, the device 100 may determine, among the plurality of standard pre-aligners, one standard pre-aligner expected to be suitable for the oral cavity of the user by comparing the oral information corresponding to the tooth shape, dentition, and the like of the user with the specification information corresponding to the arch lengths, arch widths, and tooth widths of the plurality of standard pre-aligners.

In operation S240, the device 100 according to an embodiment obtains a tooth model corresponding to the oral information.

In an embodiment, the tooth model is a model corresponding to the oral cavity of the user, and may refer to a tooth model in a state desired by the user or a tooth model determined according to the oral information of the user.

In operation S250, the device 100 according to an embodiment obtains a clear aligner corresponding to the tooth model by deforming the determined standard pre-aligner to correspond to the tooth model.

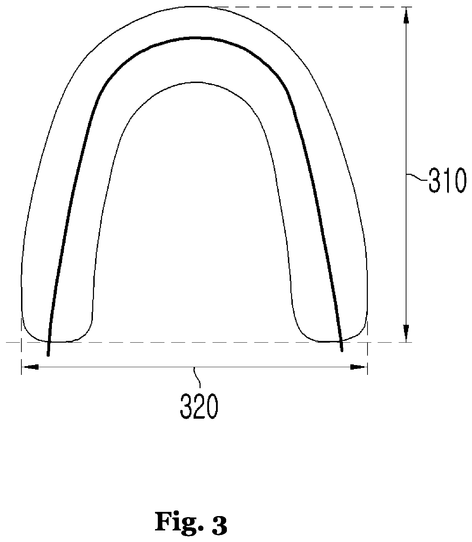

In an embodiment, the device 100 may deform the determined standard pre-aligner, among the plurality of standard pre-aligners corresponding to the plurality of preformed appliances, to correspond to the tooth model. For example, in a state in which the determined standard pre-aligner and the tooth model are coupled to each other, the device 100 may perform heat treatment and vacuum treatment on the standard pre-aligner by controlling the vacuum heat treatment unit 130. Accordingly, after performing the heat treatment and the vacuum treatment, the device 100 may separate the clear aligner corresponding to the tooth model from the tooth model. In an embodiment, an example of determining one of the plurality of standard pre-aligners and an example of obtaining the clear aligner will be described with reference to FIGS. 3 to 6.

FIG. 3 is a diagram for explaining an example in which the device 100 obtains an arch length and an arch width for a plurality of standard pre-aligners according to an embodiment.

Referring to FIG. 3, the device 100 according to an embodiment may obtain an arch length 310 and an arch width 320, which indicate specification information, for each of the plurality of standard pre-aligners. That is, as illustrated in FIG. 3, the device 100 may obtain the arch length 310 indicating an anteroposterior length of a dental arch of each of the plurality of standard pre-aligners and the arch width 320 indicating a left-right width of the dental arch.

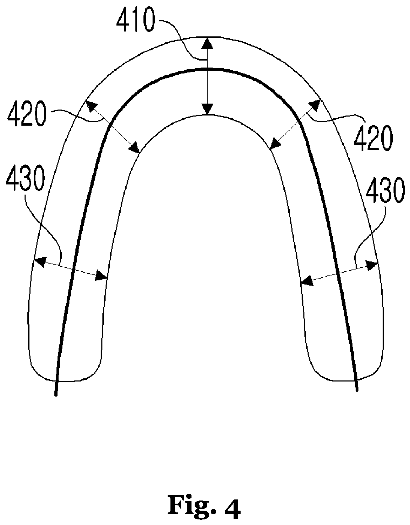

FIG. 4 is a diagram for explaining an example in which the device 100 obtains tooth widths for a plurality of standard pre-aligners according to an embodiment.

Referring to FIG. 4, the device 100 according to an embodiment may obtain tooth widths for respective sections, which indicate widths of teeth. Specifically, the device 100 may obtain a first anterior tooth width 410 and a second anterior tooth width 420 corresponding to an anterior region, and may obtain a posterior tooth width 430 corresponding to a posterior region.

In an embodiment, the first anterior tooth width 410 may be a tooth width corresponding to front teeth, namely central incisors and lateral incisors, and the second anterior tooth width 420 may be a tooth width corresponding to canine teeth located at third positions on both sides from the front teeth. In addition, the posterior tooth width 430 may be a tooth width corresponding to any one of teeth located at fourth or subsequent positions on both sides from the front teeth.

In an embodiment, a range of anterior tooth widths of the plurality of standard pre-aligners may be determined to be within a range of 20 mm to 24 mm, and a range of posterior tooth widths may be determined to be within a range of 22 mm to 25 mm.

In an embodiment, the device 100 may obtain user arch length information, user arch width information, and user tooth width information for the user based on the oral information. The device 100 may determine one corresponding standard pre-aligner among the plurality of standard pre-aligners based on the user arch length information, the user arch width information, and the user tooth width information. That is, the device 100 may determine one standard pre-aligner that most closely corresponds to the oral information of the user by comparing the user arch length information, the user arch width information, and the user tooth width information with specification information of each of the plurality of standard pre-aligners.

The user tooth width may include a tooth width of a user anterior region and a tooth width of a user posterior region. That is, the user tooth width may include tooth widths at positions corresponding to the first anterior tooth width 410, the second anterior tooth width 420, and the posterior tooth width 430 for the plurality of standard pre-aligners described above.

Accordingly, from among one or more standard pre-aligners having the arch length 310 and the arch width 320 similar to the user arch length information and the user arch width information, the device 100 may determine one standard pre-aligner that most closely corresponds to the user by respectively comparing the tooth width of the user anterior region and the tooth width of the user posterior region with the first anterior tooth width 410, the second anterior tooth width 420, and the posterior tooth width 430 of the plurality of standard pre-aligners.

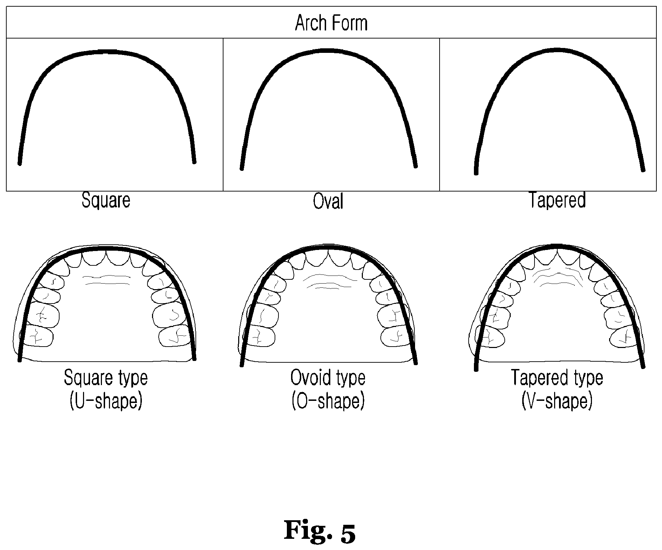

FIG. 5 is a diagram for explaining an example in which the device 100 determines one of a plurality of standard pre-aligners based on a user arch type according to an embodiment.

Referring to FIG. 5, in an embodiment, the arch type may be one of a U shape indicating a square type, an O shape indicating an ovoid type, and a V shape indicating a tapered type. The device 100 according to an embodiment may determine the user arch type as one of the U shape, the O shape, and the V shape.

The device 100 may determine one of the plurality of standard pre-aligners based on the determined user arch type. For example, the device 100 may determine, among at least one standard pre-aligner corresponding to the user arch length information, the user arch width information, and the user tooth width information, a standard pre-aligner corresponding to the user arch type. Accordingly, a standard pre-aligner to which information on the arch type of the user is applied together with the oral information may be determined as a standard pre-aligner corresponding to the user.

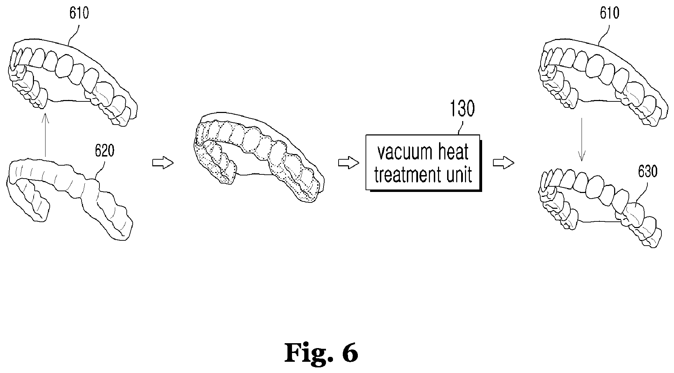

FIG. 6 is a diagram for explaining an example in which a clear aligner 630 is obtained from a three-dimensional shape according to an embodiment.

Referring to FIG. 6, the device 100 according to an embodiment may deform one of the plurality of standard pre-aligners indicating preformed appliances to correspond to a tooth model 610 of a user. The tooth model 610 of the user is generated to have the same shape as a tooth shape according to an oral structure of the user. For example, the oral structure of the user may be scanned using a 3D scanner, and the tooth model 610 of the user may be generated based on scan data. In this regard, a 3D printer may be used. The tooth model 610 of the user may be printed by outputting scan data obtained using the 3D scanner through the 3D printer.

In an embodiment, the device 100 may deform a standard pre-aligner 620, determined as one of the plurality of standard pre-aligners, to correspond to the shape of the tooth model 610 of the user by controlling the vacuum heat treatment unit 130. Accordingly, the device 100 may obtain a clear aligner 630 into which the determined standard pre-aligner 620 is deformed.

In an embodiment, the device 100 may generate a 3D image based on statistical information and may print the plurality of standard pre-aligners through a 3D printer. For example, shapes of the plurality of standard pre-aligners are formed based on 3D shapes of a plurality of standard tooth models determined based on the statistical information. A shape of any one standard pre-aligner among the plurality of standard pre-aligners may be a shape corresponding to one of the plurality of standard tooth models.

For example, the standard tooth model may be a tooth model obtained according to statistical information on dental specifications and sizes of Asians. The shape of the standard pre-aligner may be a shape coupled to each of one or more standard tooth models representing characteristics such as various dimensions and various heights, which are classified according to a standard specification range indicating dental specifications or sizes of Asians. For example, the dental specifications and sizes of Asians may be classified, and the plurality of standard pre-aligners corresponding to shapes to be coupled to a preset number, for example, nine, of standard tooth models may be obtained using dimensions, heights, and the like.

In an embodiment, an internal space of the standard pre-aligner shape may be configured to define a clearance that is maintained even after the standard tooth model is coupled to the standard pre-aligner.

Referring to FIG. 6, an example in which the determined standard pre-aligner 620 is coupled to the tooth model 610 of the user corresponding to the oral information of the user can be identified. As illustrated in a first operation of FIG. 6, the determined standard pre-aligner 620 may be coupled to the tooth model 610 of the user.

Accordingly, the device 100 may obtain the clear aligner 630 corresponding to the oral information of the user by controlling the vacuum heat treatment unit 130 to perform heat treatment and vacuum treatment on the determined standard pre-aligner 620 in a state in which the determined standard pre-aligner 620 is coupled to the tooth model 610 of the user. That is, the vacuum heat treatment unit 130 may perform the heat treatment and the vacuum treatment on the determined standard pre-aligner 620 coupled to the tooth model 610 of the user, and then the determined standard pre-aligner 620 may be separated from the tooth model 610 of the user. Accordingly, the clear aligner 630, into which the determined standard pre-aligner 620 is deformed to correspond to the tooth model 610 of the user through the heat treatment and the vacuum treatment, may be obtained.

In an embodiment, an internal space indicating a region in which the standard pre-aligner is coupled to the tooth model may be configured to define a clearance that is maintained even after the standard pre-aligner is coupled to the standard tooth model. For example, a shape of the standard pre-aligner may be determined to have a size larger by a predetermined degree so as to define the clearance from a shape of the standard tooth model generated according to a standard specification range. That is, the plurality of standard pre-aligners corresponding to a preset number, for example, nine, may each have a shape such that an internal space of each standard pre-aligner defines a clearance exceeding a region of a shape of each of the plurality of standard tooth models.

Accordingly, heat treatment and vacuum treatment may be performed in a state in which the standard pre-aligner 620 determined as one of the plurality of standard pre-aligners is coupled to the tooth model 610 of the user corresponding to the oral information of the user. Therefore, as illustrated in a final operation of FIG. 6, the clear aligner 630 corresponding to the shape of the tooth model 610 of the user may be obtained.

According to the present invention, since heat treatment and vacuum treatment are performed by coupling a preset number of standard pre-aligners to the tooth model 610 of the user, it is possible to prevent a thickness change that may occur during a heat treatment process when the clear aligner 630 is obtained using a conventional transparent sheet. That is, according to the present invention, since the plurality of standard pre-aligners indicating preformed appliances are used, a 3D model can be obtained from a 3D model, and thus it is advantageous in that a uniform thickness can be maintained.

In addition, when the clear aligner 630 is obtained using a conventional transparent sheet, a process of cutting and polishing a portion of the sheet is required. In contrast, according to the present invention, since the determined standard pre-aligner 620 exists as a 3D shape corresponding to the clear aligner 630, it is advantageous in that a polishing process is unnecessary.

The above-described embodiments are merely examples and should not be construed as limiting.

The order and combination of the operations illustrated above are merely examples, and it will be understood that the operations may be variously implemented in forms in which an order, a combination, a branch, a function, and a performing subject thereof are added, omitted, or modified within a range that does not depart from essential characteristics of each component described in the specification. In addition, throughout the specification, the term "provide" may be interpreted as including a process in which a subject obtains specific information or directly or indirectly transmits and receives specific information to and from a specific target, and as comprehensively including performance of related operations required in such a process.

Various embodiments of the present disclosure may be implemented as software including one or more instructions stored in a storage medium, for example, a memory, readable by a machine, for example, a display apparatus or a computer. For example, a processor of the machine (e.g., the processor 120) may call at least one instruction among the one or more instructions stored in the storage medium and execute the at least one instruction. This enables the machine to be operated to perform at least one function according to the called at least one instruction. The one or more instructions may include code generated by a compiler or code executable by an interpreter. The machine-readable storage medium may be provided in the form of a non-transitory storage medium. Here, the term "non-transitory" merely means that the storage medium is a tangible device and does not include a signal, for example, an electromagnetic wave, and this term does not distinguish between a case in which data is semi-permanently stored in the storage medium and a case in which data is temporarily stored in the storage medium.

According to an embodiment, a method according to various embodiments disclosed in the present disclosure may be provided as being included in a computer program product. The computer program product may be distributed in the form of a machine-readable storage medium, or may be distributed online, for example, downloaded or uploaded, through an application store, for example, Play Store^{™}, or directly between two user devices, for example, smartphones. In the case of online distribution, at least a portion of the computer program product may be at least temporarily stored in, or temporarily generated in, a machine-readable storage medium, such as a memory of a server of a manufacturer, a server of an application store, or a relay server.

It will be understood by those skilled in the art related to the present embodiment that the present disclosure may be implemented in modified forms without departing from the essential characteristics of the above description. Therefore, the disclosed methods should be considered from an illustrative viewpoint rather than a restrictive viewpoint. The scope of the present disclosure is defined by the claims rather than the foregoing description, and all differences within a scope equivalent thereto should be construed as being included in the present disclosure.

## Claims

1. A method of manufacturing a clear aligner, the method comprising:
obtaining, by a processor, specification information on a plurality of standard pre-aligners generated based on statistical information;
obtaining, by a receiver, oral information of a user;
determining, by the processor, one of the plurality of standard pre-aligners based on the oral information and the specification information;
obtaining, by the processor, a tooth model corresponding to the oral information; and
obtaining, by the processor, a clear aligner corresponding to the tooth model by deforming the determined standard pre-aligner to correspond to the tooth model.

2. The method of claim 1,
wherein obtaining the clear aligner comprises:
performing heat treatment and vacuum treatment in a state in which the determined standard pre-aligner and the tooth model are coupled to each other; and
separating the clear aligner from the tooth model after performing the heat treatment and the vacuum treatment.

3. The method of claim 1,
wherein the specification information includes information on an arch length indicating an anteroposterior length of a dental arch, an arch width indicating a left-right width of the dental arch, and a tooth width indicating a width of a tooth.

4. The method of claim 3,
wherein the tooth width includes an anterior tooth width and a posterior tooth width.

5. The method of claim 1,
wherein determining one of the plurality of standard pre-aligners based on the oral information and the specification information comprises:
obtaining user arch length information, user arch width information, and user tooth width information for the user based on the oral information; and
determining one of the plurality of standard pre-aligners based on the user arch length information, the user arch width information, and the user tooth width information.

6. The method of claim 5,
wherein determining one of the plurality of standard pre-aligners based on the user arch length information, the user arch width information, and the user tooth width information comprises:
determining, by the processor, a user arch type as one of a U shape, an O shape, and a V shape; and
determining one of the plurality of standard pre-aligners based on the determined user arch type.

7. The method of claim 1,
further comprising:
generating a 3D image based on the statistical information and printing the plurality of standard pre-aligners through a 3D printer,
wherein shapes of the plurality of standard pre-aligners are formed based on 3D shapes of a plurality of standard tooth models determined based on the statistical information.

8. The method of claim 7,
wherein an internal space of the standard pre-aligner is configured to define a clearance that is maintained even after the standard tooth model is coupled to the standard pre-aligner.

9. The method of claim 7,
wherein a shape of any one standard pre-aligner among the plurality of standard pre-aligners is a shape corresponding to one of the plurality of standard tooth models.

10. A device for manufacturing a clear aligner, the device comprising:
a receiver configured to obtain oral information of a user; and
a processor configured to:
obtain specification information on a plurality of standard pre-aligners generated based on statistical information;
determine one of the plurality of standard pre-aligners based on the oral information and the specification information;
obtain a tooth model corresponding to the oral information; and
obtain a clear aligner corresponding to the tooth model by deforming the determined standard pre-aligner to correspond to the tooth model.

11. The device of claim 10,
wherein the processor is configured to:
control a vacuum heat treatment unit to perform heat treatment and vacuum treatment in a state in which the determined standard pre-aligner and the tooth model are coupled to each other; and
separate the clear aligner from the tooth model after performing the heat treatment and the vacuum treatment.

12. The device of claim 10,
wherein the specification information includes information on an arch length indicating an anteroposterior length of a dental arch, an arch width indicating a left-right width of the dental arch, and a tooth width indicating a width of a tooth, and
wherein the tooth width includes an anterior tooth width and a posterior tooth width.

13. The device of claim 10,
wherein the processor is configured to:
obtain user arch length information, user arch width information, and user tooth width information for the user based on the oral information; and
determine one of the plurality of standard pre-aligners based on the user arch length information, the user arch width information, and the user tooth width information.

14. The device of claim 13,
wherein the processor is configured to:
determine a user arch type as one of a U shape, an O shape, and a V shape; and
determine one of the plurality of standard pre-aligners based on the determined user arch type.

15. A computer-readable recording medium having recorded thereon a program for executing the method of claim 1 on a computer.
